# EUROPEAN PATENT APPLICATION

(11) **EP 3 739 063 A1**
(43) Date of publication of application: **18.11.2020**
(21) Application number: 19737984.5
(22) Date of filing: 07.01.2019
(51) Int. Cl.: C12Q 1/6818, C12Q 1/6876

(54) **FLUORESCENT NUCLEIC ACID NANOSTRUCTURE-GRAPHENE BIOSENSOR FOR NUCLEIC ACID DETECTION**

(30) Priority: 12.01.2018 KR 20180004571
(71) Applicant: PROGENEER INC., Guro-gu Seoul 08380 (KR)
(72) Inventor: UM, Soong-Ho, Seoul 06670 (KR); KIM, Jeonghun, Hwaseong-si Gyeonggi-do 18436 (KR); AHN, Myung-Ju, Seoul 05672 (KR)
(74) Representative: Ladendorf, Oliver
(86) International application number: PCT/KR2019/000202
(87) International publication number: WO 2019/139312

(57) **Abstract**

The present invention relates to a graphene oxide complex having attached thereto a structure composed of single-stranded probes, each comprising a nucleic acid complementary to a target nucleic acid and a fluorescent substance. The graphene oxide complex can detect and quantify multiple biomarkers (target nucleic acids) in real time with high sensitivity at low cost without PCR, and thus can easily diagnose most diseases in which multiple biomarkers are involved, and evaluate the effect of drug use.

<110>    Research and Business Foundation SUNGKYUNKWAN UNIVERSITY

<120>    GRAPHENE BIOSENSOR WITH FLUORESCENCE-LABELED NUCLEIC ACID
         NANOSTRUCTURE FOR DETECTING NUCLEIC ACID

<130>    PN1710-398

<160>    14

<170>    KoPatentIn 3.0

<210>    1
<211>    39
<212>    DNA
<213>    Artificial Sequence

<220>
<223>    U1


<400>    1
aggctgattc ggttcatgcg gatccactta cggcgaatg                              39


<210>    2
<211>    76
<212>    DNA
<213>    Artificial Sequence

<220>
<223>    S1


<400>    2
ccgatgaaca ccgaatcagc ctgcgaacga ccagtttggc ccgcccaaaa tctatcacca       60

gaaaaaaaaa aaaaaa                                                       76


<210>    3
<211>    70
<212>    DNA
<213>    Artificial Sequence

<220>
<223>    S2


<400>    3
gtaggtacct ggatccgcat gagttcatcg gcatgagctg catgatgagc tggcagttga       60

gctggtgata                                                              70


<210>    4
<211>    61
<212>    DNA
<213>    Artificial Sequence

<220>
<223>    S3

<400>    4
gtcgttcgcc attcgccgta agggtaccta ctttcggaga tgtcttgata gcctcaactg    60

c                                                                    61


<210>    5
<211>    39
<212>    DNA
<213>    Artificial Sequence

<220>
<223>    U1'


<400>    5
aggctgattc ggttcatgcg gatccactta cggcgaatg                           39


<210>    6
<211>    92
<212>    DNA
<213>    Artificial Sequence

<220>
<223>    S1'


<400>    6
acggttgtac gtactaagct cagtctattc aacatcagtc tgataagcta gacaccggaa    60

ccaagacagc cacacggcga caacagaagg ta                                  92


<210>    7
<211>    92
<212>    DNA
<213>    Artificial Sequence

<220>
<223>    S2'


<400>    7
tacttaccgt gtctgtgttg gcctgttcaa ctatacaatc tactacctca taccttccgc    60

cggaacgccg gaacaagaag aacaagtagc cg                                  92


<210>    8
<211>    92
<212>    DNA
<213>    Artificial Sequence

<220>
<223>    S3'


<400>    8
ggacgaactt catatcgcat gtacaggatc atcattacca ggcagtatta cggctaccgg    60

accagcacca aggcgagaac aagaacggtg tc                                  92

<210>    9
<211>    21
<212>    DNA
<213>    Artificial Sequence

<220>
<223>    c1


<400>    9
gattttgggc gggccaaact g                                                21


<210>    10
<211>    21
<212>    DNA
<213>    Artificial Sequence

<220>
<223>    c2


<400>    10
cagctcatca tgcagctcat g                                                21


<210>    11
<211>    21
<212>    DNA
<213>    Artificial Sequence

<220>
<223>    c3


<400>    11
gctatcaaga catctccgaa a                                                21


<210>    12
<211>    22
<212>    RNA
<213>    Artificial Sequence

<220>
<223>    c1'


<400>    12
uagcuuauca gacugauguu ga                                               22


<210>    13
<211>    22
<212>    RNA
<213>    Artificial Sequence

<220>
<223>    c2'


<400>    13

ugagguagua gauuguauag uu                                                22

<210>    14
<211>    22
<212>    RNA
<213>    Artificial Sequence

<220>
<223>    c3'

<400>    14
uaauacugcc ugguaaugau ga                                                22

## Description

### [Technical Field]

The present invention relates to a graphene oxide composite having attached thereto a structure composed of single-stranded probes, each comprising a nucleic acid complementary to a target nucleic acid and a fluorescent substance.

### [Background Art]

Graphene has a planar monolayer structure of carbon atoms filling in a two-dimensional lattice, and is the basic structure of all graphite materials having different dimensional structures. That is, graphene may be the basic structure of fullerene having a zero-dimensional structure, carbon nanotubes having a one-dimensional structure, or graphite stacked in a three-dimensional structure. Many recent studies noted that graphene has unique physical properties, for example, zero bandgap, by its hexagonal crystal structure, two interpenetrating triangular sublattices, and thickness corresponding to one atom. In addition, graphene has unique electron transfer properties, and hence exhibits unique phenomena not previously observed. Examples of these phenomena include a semi-integer quantum Hall effect and a bipolar supercurrent transistor effect, which are also ascribed to the unique structure of graphene. Graphene oxide (GO), which is an oxidized form of graphene, can quench the fluorescence signal of an organic fluorescent dye through the fluorescence resonance energy transfer (FRET) phenomenon.

Meanwhile, a method of detecting a specific nucleic acid (DNA or RNA) or protein is a fundamentally important technique in scientific research. By being able to detect and identify specific nucleic acids or proteins, researchers can determine which genetic and biological markers are indicators of a person's health. The use of the method of detecting nucleic acids and proteins makes it possible to detect modification of a pathogenic gene present in a sample, or expression of a specific gene. Such molecular diagnosis is used in various fields such as infectious disease diagnosis, cancer diagnosis, genetic disease diagnosis, and personalized diagnosis in order to diagnose the root of diseases, such as DNA or RNA. Representative molecular diagnostic techniques include PCR techniques that amplify DNA within a short time (Saiki, R., et. al. Primer-directed enzymatic amplification of DNA with a thermostable DNA polymerase. Science 239, 487-91. 1998). However, a common PCR technique requires the use of an electrophoresis method to identify the amplified DNA. In order to perform such electrophoresis, a cumbersome operation of preparing an agarose gel and staining DNA with EtBr or the like has been required. In addition, a real-time PCR method, which has recently been used, does not require electrophoresis because it uses fluorescence, but has a problem in that it requires the use of expensive instruments and expensive fluorescent reagents (Higuchi, R., et. al., Kinetic PCR Analysis: Real-time Monitoring of DNA Amplification Reactions. Nature Biotechnology 11, 1026 - 1030, 1993). In recent years, Cepheid's GeneXpert system and reagents, which are PCR products for *in situ* diagnosis, have been developed and sold, but they are difficult to use in general testing applications because the system and reagents are very expensive (Helb, D., et. al., Rapid Detection of Mycobacterium tuberculosis and Rifampin Resistance by Use of On-Demand, Near-Patient Technology. J. Clin. Microbiol. 48, 229-237, 2010). Other methods include nucleic acid lateral flow assay in which identification is performed using a membrane instead of gel electrophoresis after PCR (Aveyard, J., et. al., One step visual detection of PCR products with gold nanoparticles and a nucleic acid lateral flow (NALF) device. Chem. Commun., 41, 4251-4253, 2007). However, this assay is more complicated than the gel electrophoresis technique, and hence cannot be prepared and used in the laboratory. Also, the sequence of the probe attached to the membrane has to be used so that it can specifically bind depending on the PCR amplification product. Due to these technical limitations, the use of this assay in general applications is limited. In particular, this PCR-based sequencing method is extremely vulnerable to a specific error and frequently results in serious misdiagnosis due to problems in data interpretation. For this reason, studies have been conducted to express changes, which occur when a specific nucleic acid biomarker in blood meets a sequence complementary thereto, as fluorescence, potential difference, color change or the like. However, for most of these studies, one biomarker is detected at a time, and hence it is difficult to effectively diagnose most diseases in which multiple biomarkers are involved.

In addition, since human diseases are often caused by abnormalities in multiple genes rather than abnormalities in a single gene, the accuracy and usefulness of diagnosis can be increased if multiple genes can be analyzed in a single test. Therefore, there is increasing demand for a multiplex assay platform that can be applied to diagnosis with increased sensitivity and reproducibility.

### [Disclosure]

### [Technical Problem]

It is an object of the present invention to effectively diagnose most diseases, in which multiple biomarkers are involved, by simultaneously detecting and quantifying multiple biomarkers with high sensitivity *in vitro* within a short time without a PCR analysis process, and to use the diagnostic result for patient-specific treatment.

### [Technical Solution]

To achieve the above object, the present invention provides a composition for nucleic acid detection.

The present invention also provides a composition for multiple nucleic acid detection.

The present invention also provides a method for nucleic acid detection.

The present invention also provides a method for multiple nucleic acid detection.

### [Advantageous effects]

According to the present invention, graphene oxide having attached thereto a structure composed of single-stranded probes, each comprising a nucleic acid complementary to a target nucleic acid and a fluorescent substance, can detect and quantify multiple biomarkers (target nucleic acids) in real time with high sensitivity at low costs without PCR, and thus can easily diagnose most diseases in which multiple biomarkers are involved, and evaluate the effect of drug use.

### [Description of Drawings]

FIG. 1 is a diagram showing a development model of a tetrahedral fluorescent nucleic acid nanostructure bound to target sequences.
FIG. 2 shows the results of confirming the synthesis of tetrahedral fluorescent nucleic acid nanostructures:
   Lanes A to G: products obtained by mixing strands in possible combinations of one to three single-stranded probes, followed by heat treatment; and
   Lane H (t-DNA): a product (tetrahedral fluorescent nucleic acid nanostructure) obtained by mixing all four single-stranded probes.
FIG. 3 is a diagram showing a development model of a triangular prism-shaped fluorescent nucleic acid nanostructure bound to target sequences.
FIG. 4 is a diagram showing the design and simulation results of a tetrahedral fluorescent nucleic acid nanostructure:
   a: the design of the tetrahedral fluorescent nucleic acid nanostructure; and
   b: a fluorescence emission model when a tetrahedral fluorescent nucleic acid nanostructure/graphene oxide complex binds to target sequences.
FIG. 5 is a diagram showing the design and simulation results of a triangular prism-shaped fluorescent nucleic acid nano-probe:
   a: fluorescence emission models when a triangular prism-shaped fluorescent nucleic acid nanostructure/graphene oxide complex binds to target sequences;
   b: the results of a simulation performed using the oxDNA program; and
   c: the results of predicting the distance between fluorescence and graphene oxide.

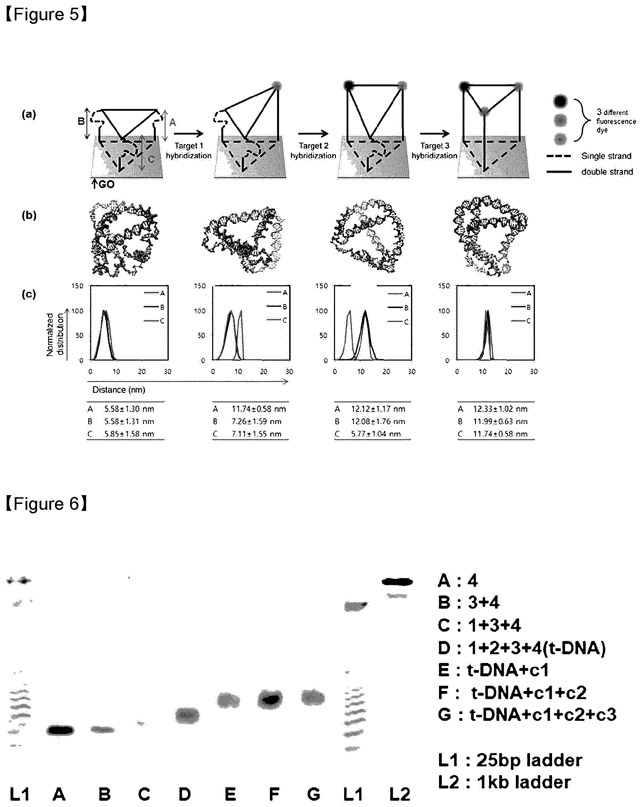

FIG. 6 shows the results of synthesizing tetrahedral fluorescent nucleic acid nanostructures and the results of producing tetrahedral fluorescent nucleic acid nanostructures by mixing with target sequences:
   Lanes A to C: a combination of four probes;
   Lane D (t-DNA): a product (tetrahedral fluorescent nucleic acid nanostructure) obtained by mixing all four probes, followed by heat treatment; and
   Lanes E to G: products obtained by mixing the tetrahedral fluorescent nucleic acid nanostructure with target sequences (c1, c2 and c3), followed by heat treatment.

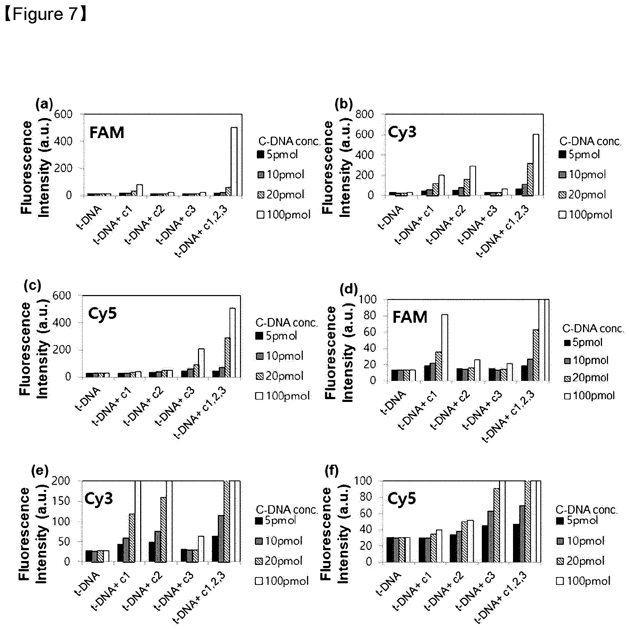

FIG. 7 shows the target nucleic acid biomarker detection range of a tetrahedral fluorescent nucleic acid nanostructure/graphene oxide complex produced by adding target sequences:
   a to c: the results of measuring fluorescence intensities corresponding to the absorption/emission wavelengths of FAM (485 nm/525 nm), Cy3 (540 nm/570 nm) and Cy5 (640 nm/670 nm) for the tetrahedral fluorescent nucleic acid nanostructure/graphene oxide complex produced by synthesizing the fluorescent nucleic acid nanostructure through adding target sequences (c1, c2 and c3) at a molar ratio of 0 to 5 times (5, 10, 20 and 100 pmol) during synthesis of the fluorescent nucleic acid nanostructure; and
   d to f: graphs obtained by enlarging a to c to certain values.

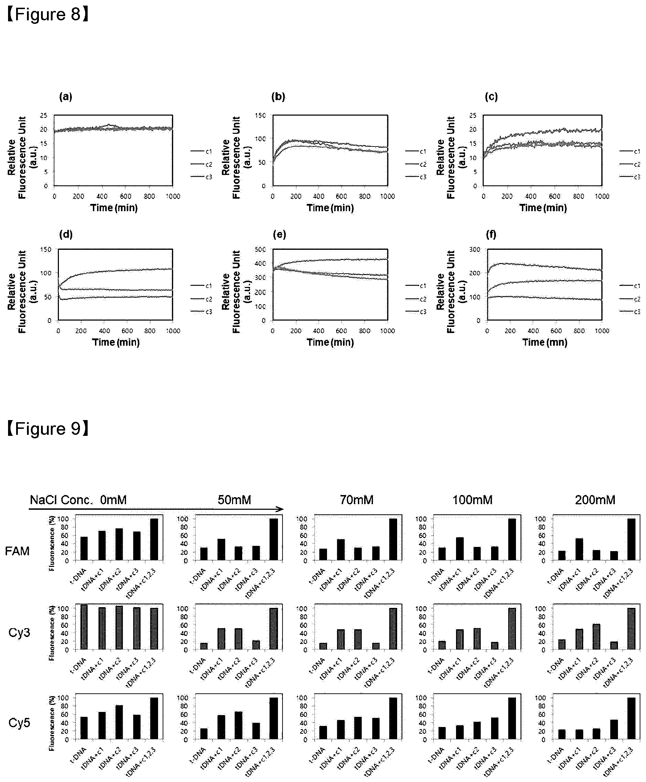

FIG. 8 shows the biomarker detection ranges when target sequences are added after forming tetrahedral fluorescent nucleic acid nanostructure/graphene oxide complex:
   a to c: the results of measuring fluorescence when 20 pmol/10 µl of a target sequence (c-DNA) reacted with 20 pmol/50 µl of the tetrahedral fluorescent nucleic acid nanostructure/graphene oxide complex;
   d to f: the results of measuring fluorescence when 200 pmol/10 µl of a target sequence (c-DNA) reacted with 20 pmol/50 µl of the tetrahedral fluorescent nucleic acid nanostructure/graphene oxide complex;
   a and d: FAM (485 nm/525 nm);
   b and e: Cy3 (540 nm/570 nm); and
   c and f: Cy5 (640 nm/670 nm).
FIG. 9 shows the target nucleic acid biomarker detection ability of a tetrahedral fluorescent nucleic acid nanostructure/graphene oxide complex depending on the concentration of salt (NaCl).

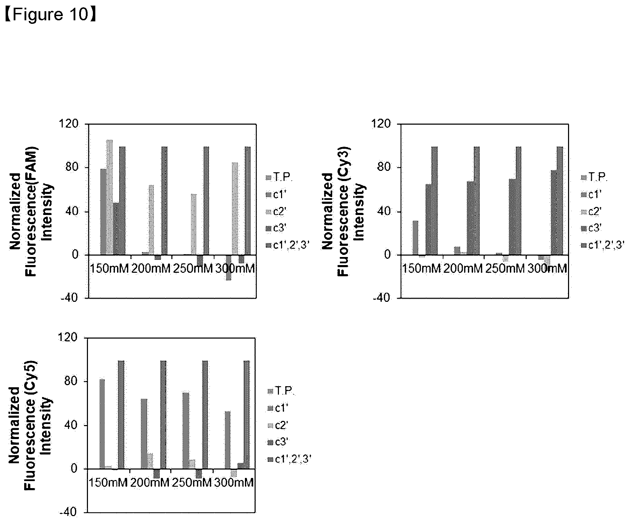

FIG. 10 shows the target nucleic acid biomarker detection ability of a triangular prism-shaped fluorescent nucleic acid nanostructure/graphene oxide complex depending on the concentration of salt (NaCl) used in production of the triangular prism-shaped fluorescent nucleic acid nanostructure.

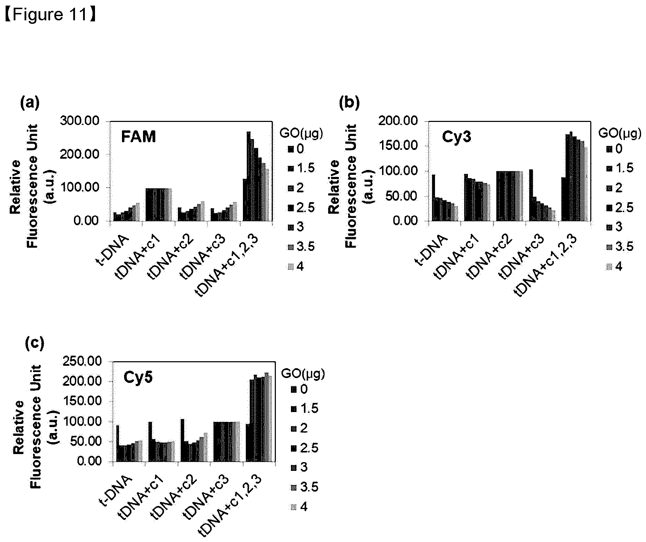

FIG. 11 shows the target nucleic acid biomarker detection abilities of tetrahedral fluorescent nucleic acid nanostructure/graphene oxide complexes depending on the amount of graphene oxide.
   t-DNA: a tetrahedral fluorescent nucleic acid nanostructure/graphene oxide complex to which no target was added;
   t-DNA+c1: a tetrahedral fluorescent nucleic acid nanostructure/graphene oxide complex produced by adding target sequence c1 (L858R);
   t-DNA+c2: a tetrahedral fluorescent nucleic acid nanostructure/graphene oxide complex produced by adding target sequence c2 (T790M);
   t-DNA+c3: a tetrahedral fluorescent nucleic acid nanostructure/graphene oxide complex produced by adding target sequence c3 (Del Ex19 (E746-A750)); and
   t-DNA+c1, 2, 3: a tetrahedral fluorescent nucleic acid nanostructure/graphene oxide complex produced by adding target sequences c1, c2 and c3.

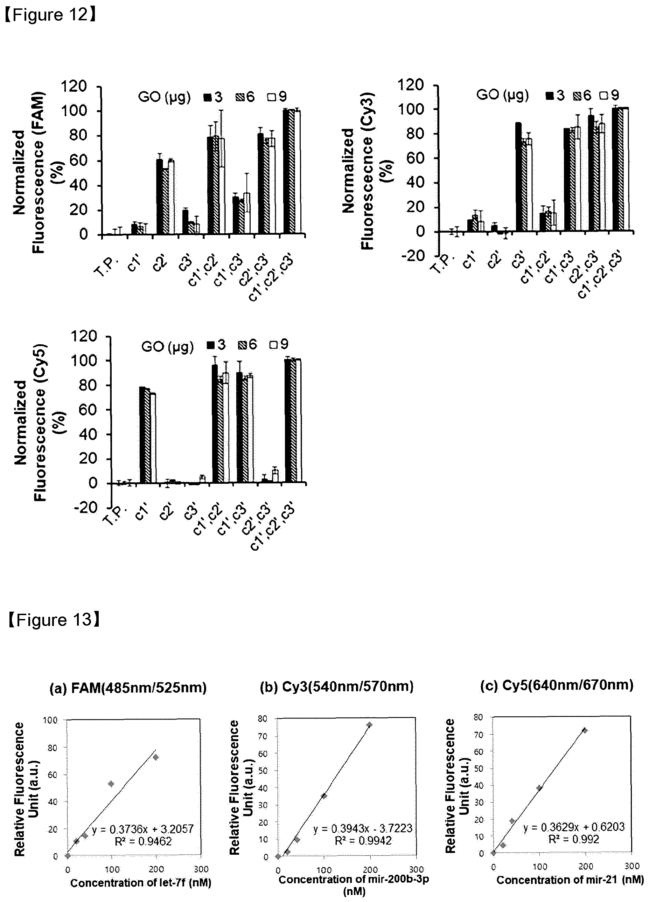

FIG. 12 shows the target nucleic acid biomarker detection abilities of triangular prism-shaped fluorescent nucleic acid nanostructure/graphene oxide complexes depending on the amount of graphene oxide:
   T.P.: a triangular prism-shaped fluorescent nucleic acid nanostructure/graphene oxide complex to which no target was added.
FIG. 13 depicts graphs showing the fluorescence value of a triangular prism-shaped fluorescent nucleic acid nanostructure/graphene oxide complex depending on the amount of each target nucleic acid biomarker.

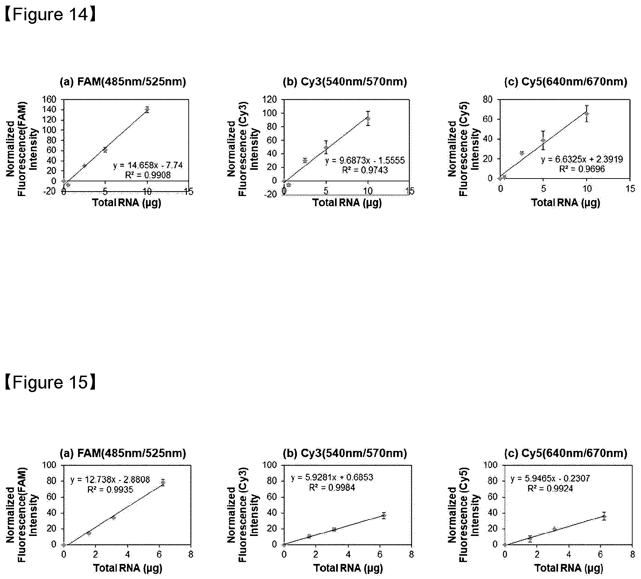

FIG. 14 depicts graphs showing the results of analyzing the detection ability of a triangular prism-shaped fluorescent nucleic acid nanostructure/graphene oxide complex for total miRNA extracted from lung cancer A549 cells.
FIG. 15 depicts graphs showing the detection ability of a triangular prism-shaped fluorescent nucleic acid nanostructure/graphene oxide complex for total miRNA extracted from lung cancer PC-9 cells.

### [Best Mode]

Hereinafter, the present invention will be described in detail with reference to embodiments of the present invention. However, the following embodiments are presented as examples of the present invention, the present invention is not limited thereby, and the present invention can be variously modified and applied within the scope of the claims and the equivalents interpreted therefrom.

Unless otherwise indicated, nucleic acids are in 5'→3' orientation from left to right. Numeric ranges recited within the specification are inclusive of the numbers defining the range and include each integer or any non-integer fraction within the defined range.

Unless defined otherwise, all technical and scientific terms used herein have the same meaning as commonly understood by those skilled in the art to which the present invention pertains. Although any methods and materials similar or equivalent to those described herein can be used for testing of the subject matter recited in the present invention, the preferred materials and methods are described herein.

As used herein, the term "fluorescent nucleic acid nanostructure" includes single-stranded probes, each comprising a nucleic acid complementary to a target nucleic acid and a fluorescent substance, a structure composed of the probes, or the probes bound to target nucleic acids.

As used herein, the term "fluorescent nucleic acid nanostructure/graphene oxide complex" or "graphene fluorescent nucleic acid nanostructure" refers to a structure or complex in which the fluorescent nucleic acid nanostructure is attached to graphene oxide.

As used herein, the term "probe" refers to a nucleic acid fragment, such as RNA or DNA, which can bind specifically to target nucleic acid biomarkers, including miRNA, mRNA and DNA, and ranges from several nucleotides to several hundred nucleotides in length. The probe can labeled to determine the presence or absence of a specific mRNA. The probe may be constructed in the form of an oligonucleotide probe, a single-stranded DNA probe, a double-stranded DNA probe, an RNA probe or the like. In the present invention, the term "probe" refers to a single-stranded nucleic acid.

Throughout the specification of the present invention, when any member is referred to as being "on" another member, it not only refers to a case where any member is in contact with another member, but also a case where a third member exists between the two members.

As used herein, the term "sample" includes tissue, cells, blood, serum, urine, saliva, plasma or body fluid obtained from a subject or patient. The source of the tissue or cell sample may be solid tissue from a fresh, frozen and/or preserved organ or tissue sample or biopsy or aspirate; blood or any blood constituents; or cells from any time in gestation or development of the subject. The tissue sample may also be primary or cultured cells or cell lines.

As used herein, the term "detection" or "measurement" refers to quantifying the concentration of a detected or measured subject.

In one aspect, the present invention is directed to a composition for nucleic acid detection comprising: a single-stranded probe comprising a nucleic acid complementary to a target nucleic acid and a fluorescent substance; and graphene oxide.

In one embodiment, the single-stranded probe may be DNA, RNA, or DNA and RNA.

In one embodiment, the composition may comprise one or more single-stranded probes, each comprising a nucleic acid complementary to a target nucleic acid. When the number of single-stranded probes, each comprising a nucleic acid complementary to a target nucleic acid, is 3 or more, a three-dimensional structure may be formed by further including a single-stranded probe consisting of sequences complementary to some sequences except the nucleic acid region complementary to the target nucleic acid of each single-stranded probe. In addition, there are advantages in that multiplex detection of different target nucleic acids is possible by including one or more single-stranded probes, each comprising a nucleic acid complementary to a different target nucleic acid and a different fluorescent substance, and the detection results are stably maintained due to the three-dimensional structure, and it is possible to quantify multiple target nucleic acids, respectively.

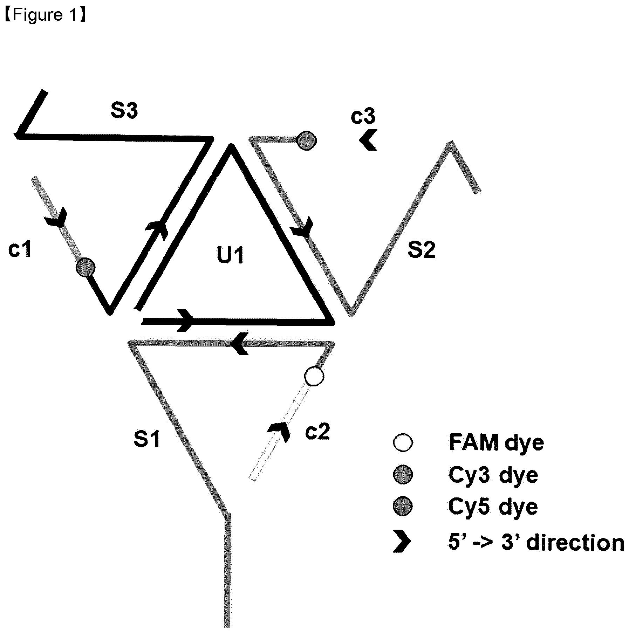

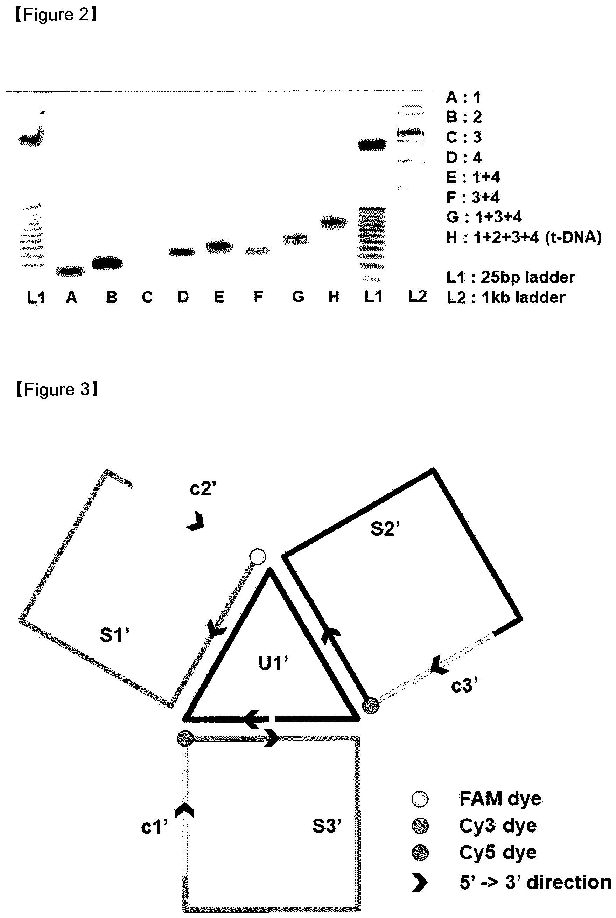

In one embodiment, the fluorescent nucleic acid nanostructure of the present invention comprises: sequence regions complementary to target sequences (c1, c2 and c3/c1', c2' and c3'); fluorescent substances; three single-stranded probes (S1, S2 and S3/S1', S2' and S3'), each comprising a sequence region constituting the structure; and a probe (U1/U1') that binds complementarily to the three probes to form double strands. In one example of the present invention, tetrahedral and triangular prism-shaped fluorescent nucleic acid nanostructures capable of detecting three different target nucleic acids were produced. One side of the tetrahedral nanostructure consists of a double strand, and a portion of each lateral side (a portion to which a target sequence binds complementarily) and a portion that is attached to graphene oxide consist of a single strand. The upper side of the triangular prism-shaped nanostructure consists of a double strand, and a portion of each lateral side (a portion to which a target sequence binds complementarily) and the bottom that is attached to graphene oxide consist of a single strand. Specifically, when there are no target nucleic acids (sequences), that is, c1, c2 and c3, in the tetrahedral fluorescent nucleic acid nanostructure, all portions of the structure, except portions complementary to the targets of S1, S2 and S3 and the poly-A tail portion to be attached to graphene oxide, form a three-dimensional structure by forming double bonds (junctions); and when a fluorescent nucleic acid nanostructure comprising the target nucleic acids c1, c2 and c3 is formed, the target nucleic acids c1, c2 and c3 bind to the complementary single-stranded sequence portions of S1, S2 and S3, respectively, and thus all portions of the structure, except the poly-A tail portion, form double bonds (see FIG. 1). When there are no target nucleic acids (sequences), that is, c1', c2' and c3', in the triangular prism-shaped fluorescent nucleic acid nanostructure, all portions of the structure, except the target-complementary portions of S1', S2' and S3' and the triangular bottom of the triangular prism that binds to graphene oxide, form a three-dimensional structure by junctions forming double bonds; and when a fluorescent nucleic acid nanostructure comprising the target nucleic acids c1', c2' and c3' is formed, the target nucleic acids c1', c2' and c3' bind to the complementary sequences of S1', S2' and S3', respectively, and thus all portions of the structure, except one triangular side of the triangular prism that is to be attached to graphene oxide, form double bonds (see FIG. 3).

In one embodiment, since the sequence of the target nucleic acid-complementary portion of each probe may vary depending on the target, nucleic acids specific for a particular disease may be selected as targets and used for diagnosis of the disease. In one example of the present invention, c1, c2 and c3, which are modified EGFR mRNA sequences specific to lung cancer, and c1', c2' and c3' which are miRNA sequences, were used as target sequences, and thus these target sequences could be used to diagnose lung cancer and monitor the effect of use of an anticancer drug.

The probes of the present invention may be chemically synthesized using a phosphoramidite solid support method or other well-known methods. Such nucleic acid sequences may also be modified using a number of means known in the art. Non-limiting examples of such modifications include methylation, capsulation, substitution of one or more native nucleotides with analogues thereof, and inter-nucleotide modifications, for example, modifications to uncharged conjugates (e.g., methyl phosphonate, phosphotriester, phosphoramidate, carbamate, etc.) or charged conjugates (e.g., phosphorothioate, phosphorodithioate, etc.).

In one embodiment, one or more single-stranded probes comprising nucleic acids complementary to different target nucleic acids and different fluorescent substances may be assembled into a fluorescent nucleic acid nanostructure through a specific heat treatment process.

In one embodiment, the heat treatment may differ depending on the structure of the fluorescent nucleic acid nanostructure. When the fluorescent nucleic acid nanostructure is tetrahedral, the heat treatment may be performed under the following conditions: 95°C for 2 min; temperature lowering from 60°C at a rate of 1°C/min; and 20°C for 5 min and temperature lowering to 4°C. When the fluorescent nucleic acid nanostructure is a triangular prism, the heat treatment may be performed under the following conditions: 95°C for 5 min; 85°C for 5 min; temperature lowering at a rate of 0.5°C/min; and 20°C for 5 min and temperature lowering to 4°C.

In one embodiment, graphene oxide (GO) is a universal quencher (UQ) that can selectively quench fluorescence by fluorescence resonance energy transfer. When a target nucleic acid and the single-stranded probe of the present invention bind to each other to form a double bond, the distance between the probe (or the fluorescent nucleic acid nanostructure) and the graphene oxide increases, and thus luminescence of the fluorescent substance can be detected. That is, graphene oxide has a unique property of more easily attaching to a single-stranded sequence than a double-stranded sequence, and when the distance between graphene oxide and the fluorescent substance of the fluorescent nucleic acid nanostructure is low, fluorescence is quenched by fluorescence resonance energy transfer, and when the distance increases, fluorescence can be detected again. Thus, when multiple target nucleotide sequences bind to sequences complementary to the probes comprising the fluorescent substances to form double bonds, the distance from the graphene oxide increases, so that fluorescence is emitted. Based on these properties, in one example of the present invention, a complex was designed so that it could recognize and quantify multiple targets at the same time.

In another aspect, the present invention is directed to a composition for nucleic acid detection which fluorescent nucleic acid nanostructure is attached to graphene oxide, wherein the fluorescent nucleic acid nanostructure comprises: a first single-stranded probe comprising a nucleic acid complementary to a first target nucleic acid and a fluorescent substance, in which a portion of the single strand is attached to graphene oxide; a second single-stranded probe comprising a nucleic acid complementary to a second target nucleic acid and a fluorescent substance; a third single-stranded probe comprising a nucleic acid complementary to a third target nucleic acid and a fluorescent substance; and a fourth single-stranded probe composed of a sequence complementary to the first single-stranded probe, a sequence complementary to the second single-stranded probe, and a sequence complementary to the third single-stranded probe.

In one embodiment, the fluorescent substances included in the single-stranded probes may be fluorescent substances having different wavelength bands.

In one embodiment, the fluorescent substances may be selected from among fluorescein (FAM), Texas Red, rhodamine, Alexa, cyanine (Cy), BODIPY, acetoxymethyl ester, and coumarin, but are not limited thereto, and any fluorescent substances may be used as long as they are known fluorescent substances to which single-stranded nucleic acids may be attached.

In one embodiment, the target nucleic acids may be c1, c2 and c3 represented by the nucleotide sequences of SEQ ID NOs: 9, 10 and 11, respectively; the first to third single-stranded probes may be S1, S2 and S3 represented by the nucleotide sequences of SEQ ID NOs: 2, 3 and 4, respectively, which are labeled with fluorescent substances and comprise sequences complementary to the target nucleic acids; and the fourth single-stranded probe may be U1 represented by the nucleotide sequence of SEQ ID NO: 1, which is composed of sequences complementary to portions of S1, S2 and S3 (see FIG. 1) . In addition, in one embodiment, the target nucleic acids may be c1', c2' and c3' represented by the nucleotide sequences of SEQ ID NOs: 12, 13 and 14; the first to third single-stranded probes may be single strands S1', S2' and S3' represented by the nucleotide sequences of SEQ ID NOs: 6, 7 and 8, respectively, which are labeled with fluorescent substances and comprise sequences complementary to the target nucleic acids; and the fourth single-stranded probe may be U1' represented by the nucleotide sequence of SEQ ID NO: 5, which is composed of sequences complementary to portions of S1', S2' and S3' (see FIG. 3). In addition, the target nucleic acid-complementary sequence in each probe may be modified depending on the target. In one Example of the present invention, c1, c2 and c3, which are modified EGFR mRNA sequences specific to lung cancer, and c1', c2' and c3' which are miRNA sequences, were used as target sequences, and thus these target sequences could be used to diagnose lung cancer and monitor the effect of use of an anticancer drug.

In one embodiment, one side of the tetrahedron formed by the fourth single-stranded probe may be present farthest from the graphene oxide.

In still another aspect, the present invention is directed to a composition for nucleic acid detection in which a fluorescent nucleic acid nanostructure is attached to graphene oxide, wherein the fluorescent nucleic acid nanostructure comprises: a first single-stranded probe comprising a nucleic acid complementary to a first target nucleic acid and a fluorescent substance; a second single-stranded probe comprising a nucleic acid complementary to a second target nucleic acid and a fluorescent substance; a third single-stranded probe comprising a nucleic acid complementary to a third target nucleic acid and a fluorescent substance; and a fourth single-stranded probe composed of sequences complementary to portions of the first single-stranded probe, the second single-stranded probe and the third single-stranded probe.

In one embodiment, among the triangular sides of the triangular prism, the fourth single-stranded probe and a side (single-stranded portion) opposite the double-stranded side formed by the first to third single-stranded probes may be attached to the graphene oxide.

In yet another aspect, the present invention is directed to a method for nucleic acid detection comprising steps of: isolating nucleic acid from a sample; producing a fluorescent nucleic acid nanostructure by mixing the isolated nucleic acid with a single-stranded probe comprising a nucleic acid complementary to a target nucleic acid and a fluorescent substance, followed by heat treatment; attaching the fluorescent nucleic acid nanostructure to graphene oxide; and detecting fluorescence by a fluorescence resonance energy transfer phenomenon.

In one embodiment, the concentration of salt (NaCl) in the step of producing the fluorescent nucleic acid nanostructure and the step of attaching the fluorescent nucleic acid nanostructure to the graphene oxide may be 50 to 300 mM.

In still yet another aspect, the present invention is directed to a method for nucleic acid detection comprising steps of: isolating nucleic acid from a sample; reacting the isolated nucleic acid with the composition for nucleic acid detection according to the present invention; and detecting fluorescence by a fluorescence resonance energy transfer phenomenon.

In one embodiment, the composition for nucleic acid detection according to the present invention may be a graphene/fluorescent nucleic acid nanostructure in which a fluorescent nucleic acid nanostructure is attached to graphene oxide, wherein the fluorescent nucleic acid nanostructure comprises: a first single-stranded probe comprising a nucleic acid complementary to a first target nucleic acid and a fluorescent substance; a second single-stranded probe comprising a nucleic acid complementary to a second target nucleic acid and a fluorescent substance; a third single-stranded probe comprising a nucleic acid complementary to a third target nucleic acid and a fluorescent substance; and a fourth single-stranded probe composed of sequences complementary to portions of the first single-stranded probe, the second single-stranded probe and the third single-stranded probe.

In yet another aspect, the present invention is directed to a method for multiple nucleic acid detection comprising steps of: isolating nucleic acids from a sample; producing a fluorescent nucleic acid nanostructure by mixing the isolated nucleic acids with one or more single-stranded probes comprising nucleic acids complementary to different target nucleic acids and fluorescent substances, followed by heat treatment; attaching the fluorescent nucleic acid nanostructure to graphene oxide; and detecting fluorescence by a fluorescence resonance energy transfer phenomenon.

In still another aspect, the present invention is directed to a method for producing a fluorescent nucleic acid nanostructure/graphene oxide complex, the method comprising steps of: producing a fluorescent nucleic acid nanostructure by mixing one or more single-stranded probes comprising nucleic acids complementary to different target nucleic acids and different fluorescent substances, followed by heat treatment; and attaching the fluorescent nucleic acid nanostructure to graphene oxide.

In one embodiment, the one or more single-stranded probes comprising nucleic acids complementary to different target nucleic acids and different fluorescent substances can be: a first single-stranded probe comprising a nucleic acid complementary to a first target nucleic acid and a fluorescent substance; a second single-stranded probe comprising a nucleic acid complementary to a second target nucleic acid and a fluorescent substance; a third single-stranded probe comprising a nucleic acid complementary to a third target nucleic acid and a fluorescent substance; and a forth single-stranded probe composed of sequences complementary to portions of the first single-stranded probe, the second single-stranded probe and the third single-stranded probe.

In yet another aspect, the present invention is directed to the use of a composition, which comprises one or more single-stranded probes, each comprising a nucleic acid complementary to a target nucleic acid, a fluorescent substance and a nucleic acid complementary to other single-stranded probe, and graphene oxide, for nucleic acid detection.

In yet another aspect, the present invention is directed to a method of detecting a biomarker using a composition comprising one or more single-stranded probes, each comprising a nucleic acid complementary to a target nucleic acid, a fluorescent substance and nucleic acids complementary to other single-stranded probes, and graphene oxide.

### [Mode for Invention]

The present invention will be described in more detail with reference to the following examples. However, the following examples are merely to illustrate the present invention in detail, and the present invention is not limited thereto.

### Example 1. Synthesis of Graphene Oxide

1 g of graphite and 50 g of salt (NaCl) were ground using a mortar and pestle for 15 minutes. Thereafter, to remove the salt, the ground graphite and salt were added to water, followed by stirring for 6 hours or more. The graphite was filtered using a Buchner funnel and dried in an oven at 80°C. 23 ml of 98% sulfuric acid was added to the dried graphite and stirred for 12 hours. After adding 3 g of potassium permanganate, the mixture was stirred at 35°C for 30 minutes, and then heated to 70°C, followed by stirring for 45 minutes. After adding 46 ml of water, the mixture was stirred at 98°C for 30 minutes, and then 140 ml of water and 10 ml of 30% hydrogen peroxide were added thereto, followed by stirring for 1 hour. After completion of all reactions, 30 ml of the reaction product was placed in a 50 ml tube, centrifuged at 11,000 rpm for 20 minutes, and washed once with 5% hydrochloric acid and 3 times with water. For sizing, the resulting material was centrifuged at 5,000 g for 10 minutes, and the supernatant was collected and centrifuged at 11,000 rpm for 60 minutes, and then the lower layer was collected.

### Example 2. Synthesis of Fluorescent Nucleic Acid Nanostructures

### 2-1. Synthesis of Tetrahedral Fluorescent Nucleic Acid Nanostructure

A tetrahedral fluorescent nucleic acid nanostructure was synthesized in which the bottom of the tetrahedron consisted of a double strand and the lateral sides and the portion to be attached to graphene oxide consisted of a single strand. Specifically, a total of four single strands comprises: the sequence U1 (consisting of sequences complementary to S1, S2 and S3, respectively) of the upper side of the structure (the bottom of the tetrahedron), which consists of a double strand; and S1, S2 and S3 comprising sequences complementary to the target sequences c1, c2 and c3 (modifications of EGFR mRNA), respectively (Table 1). The fluorescence-labeled nucleic acid single strands S1, S2 and S3, and U1 were quantified by measuring absorbance at a wavelength of 260 nm. Then, the strands were variously combined (combinations of one to three strands and a combination of all four strands) in the same number of moles so that the molar ratio between all the sequences was 1:1, and the concentration of salt was adjusted to 150 mM. Thereafter, each combination was placed in a polymerase chain reaction (PCR) system. Next, each combination was heat-treated under the following sequential conditions: temperature rise to 95°C and maintaining that temperature for 2 minutes; temperature lowering to 60°C; temperature lowering to 20°C at a rate of 1°C/min; maintaining at 20°C for 5 minutes; and temperature lowering to 4°C. Subsequently, the synthesis of the produced tetrahedral fluorescent nucleic acid nanostructure was confirmed by agarose gel electrophoresis. FIG. 1 shows a specific development model of the tetrahedral fluorescent nucleic acid nanostructure.

**[Table 1]**

| **Sequence name** | **Sequences (5' -> 3')** | **Length (base)** | **SEQ ID NO.** |
|---|---|---|---|
| U1 | AGG CTG ATT CGG TTC ATG CGG ATC CAC TTA CGG CGA ATG | 39 | 1 |
| S1 | CCG ATG AAC ACC GAA TCA GCC TGC GAA CGA CCA GTT TGG CCC GCC CAA AAT CTA TCA CCA GAA AAA AAA AAA AAA A | 76 | 2 |
| S2 | [?]TA GGT ACC TGG ATC CGC ATG AGT TCA TCG GCA TGA GCT GCA TGA TGA [?]CT GGC AGT TGA GCT GGT GAT A | 70 | 3 |
| S3 | GTC GTT CGC CAT TCG CCG TAA GGG TAC CTA CTT TCG GAG ATG TCT TGA TAG CCT CAA CTG C | 61 | 4 |
| c1 (L858R) | GAT TTT GGG CGG GCC AAA CTG | 21 | 9 |
| c2 (T790M) | CAG CTC ATC ATG CAG CTC ATG | 21 | 10 |
| c3 (Del Ex19, E746-A750) | GCT ATC AAG ACA TCT CCG AAA | 21 | 11 |

The fluorescent nucleic acid nanostructures (A to G), obtained by mixing strands in all possible combinations of one to three strands, and the fluorescent nucleic acid nanostructure (H) obtained by mixing all four strands were confirmed by agarose gel electrophoresis, and as a result, it was shown that the single strands were combined with each other, and thus the bands on the electrophoresis gel got gradually higher. In addition, it was confirmed that, when the four strands were heat-treated together, the nanostructure (H) was formed (FIG. 2).

### 2-2. Synthesis of Triangular Prism-Shaped Fluorescent Nucleic Acid Nanostructure

A triangular prism-shaped fluorescent nucleic acid nanostructure was synthesized in which the upper side of the triangular prism consisted of a double strand and the lateral sides and the bottom portion to be attached to graphene oxide consisted of a single strand. Specifically, a total of four single strands comprises: the sequence U1' of the upper side (a portion of the bottom of the triangular prism) of the structure, which consists of a double strand; and S1', S2' and S3' comprising sequences complementary to the target sequences c1', c2' and c3' (miRNA) (Table 2). The fluorescence-labeled single strands S1', S2' and S3', and U1' were quantified by measuring absorbance at a wavelength of 260 nm. Then, the strands were variously combined (combinations of one to three strands and a combination of all four strands) in the same number of moles so that the molar ratio between all the sequences was 1:1, and the concentration of salt was adjusted to 200 mM (the optimum salt concentration determined by additionally performing a salt concentration optimization experiment because the sequences of the triangular prism are longer and more complex than those of the tetrahedron; see Example 5-2 below). Thereafter, each combination was placed in a polymerase chain reaction (PCR) system. Next, each combination was heat-treated under the following sequential conditions: temperature rise to 95°C and maintaining at that temperature for 5 minutes; temperature lowering to 85°C and maintaining at that temperature for 5 minutes; temperature lowering to 20°C at a rate of 0.5°C/min and maintaining at that temperature for 5 minutes; and temperature lowering to 4°C. Thereafter, the synthesis of the produced triangular fluorescent prism-shaped nucleic acid nanostructure was confirmed by agarose gel electrophoresis (data not shown). FIG. 3 shows a detailed developed model of the triangular fluorescent prism-shaped nucleic acid nanostructure.

**[Table 2]**

| **Sequence name** | **Sequences (5' -> 3')** | **Length (base)** | **SEQ IN NO.** |
|---|---|---|---|
| U1' | AGG CTG ATT CGG TTC ATG CGG ATC CAC TTA CGG CGA ATG | 84 | 5 |
| S1' | ACG GTT GTA CGT ACT AAG CTC AGT CTA TTC AAC ATC AGT CTG ATA AGC TAG ACA CCG GAA CCA AGA CAG CCA CAC GGC GAC AAC AGA AGG TA | 92 | 6 |
| S2' | TAC TTA CCG TGT CTG TGT TGG CCT GTT CAA CTA TAC AAT CTA CTA CCT CAT ACC TTC CGC CGG AAC GCC GGA ACA AGA AGA ACA AGT AGC CG | 92 | 7 |
| S3' | GGA CGA ACT TCA TAT CGC ATG TAC AGG ATC ATC ATT ACC AGG CAG TAT TAC GGC TAC CGG ACC AGC ACC AAG GCG AGA ACA AGA ACG GTG TC | 92 | 8 |
| c1' (miR-21) | JAG CUU AUC AGA CUG AUG UUG A | 22 | 12 |
| c2' (let-7f) | JGA GGU AGU AGA UUG UAU AGU U | 22 | 13 |
| c3' (miR-200b-3p) | JAA UAC UGC CUG GUA AUG AUG A | 22 | 14 |

### Example 3. Formation of Fluorescent Nucleic Acid Nanostructure/Graphene Oxide Complex

### 3-1. Production of Tetrahedral Fluorescent Nucleic Acid Nanostructure/Graphene Oxide Complex

20 pmol of the tetrahedral fluorescent nucleic acid nanostructure produced in Example 2-1 above was mixed with 3 µg of the graphene oxide produced in Example 1 to reach a total volume of 50 µl, and the concentration of salt in the mixture was adjusted to 200 mM. Thereafter, the mixture was allowed to react at 25°C for 30 minutes, thereby producing a graphene/tetrahedral fluorescent nucleic acid nanostructure having a structure in which the bottom of the tetrahedron faces up and the target-complementary single-stranded portion of the lateral surface and the adenine sequence (poly A) of the S1 strand protrude and are attached to the graphene oxide.

### 3-2. Production of Triangular Prism-Shaped Fluorescent Nucleic Acid Nanostructure/Graphene Oxide Complex

10 pmol of the triangular prism-shaped fluorescent nucleic acid nanostructure produced in Example 2-2 above was mixed with 3 µg of the graphene oxide produced in Example 1, and water and salt were added thereto to reach a total volume of 50 µl, and the concentration of salt in the mixture was adjusted to 200 mM. Thereafter, the mixture was allowed to react at 25°C for 30 minutes, thereby producing a graphene/triangular prism-shaped fluorescent nucleic acid nanostructure having a structure in which the lateral sides comprise a target-complementary portion consisting of a single strand and among two triangular sides of the triangular prism, a bottom consisting of a single strand is attached to the graphene oxide.

### 3-3. Simulation of Structures of Fluorescent Nucleic Acid Nanostructure/Graphene Oxide Complexes

### 3-3-1. Tetrahedral Structure

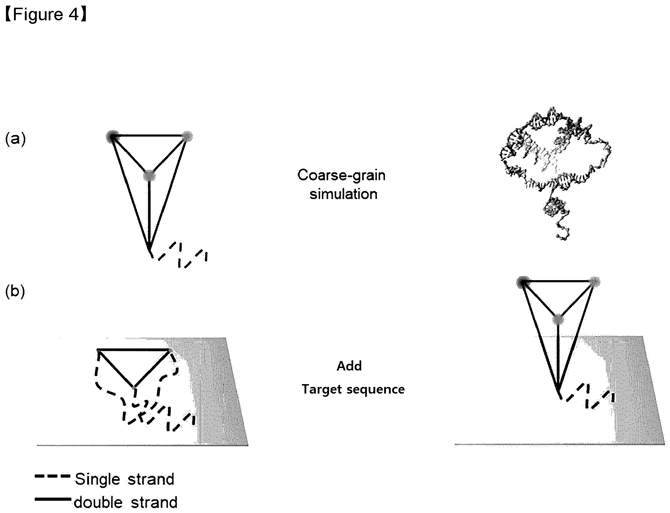

In order to examine whether the structure of the produced fluorescent nucleic acid nanostructure/graphene oxide complex was formed and to examine the final shape and the change in the position of fluorescence depending on the presence or absence of a target, a simulation was performed using the oxDNA program. Specifically, target sequences were assembled one by one into the tetrahedral fluorescent nucleic acid nanostructure, and as a result, it was shown that a double bond was formed at a predetermined position without writhe (FIGS. 4a and 4b).

### 3-3-3. Triangular Prism-Shaped Structure

In order to examine whether the structure of the produced fluorescent nucleic acid nanostructure/graphene oxide complex (graphene/triangular prism-shaped fluorescent nucleic acid nanostructure) was formed and to examine the final shape and the change in the position of fluorescence depending on the presence or absence of a target, a simulation was performed using the oxDNA program. Specifically, target sequences were assembled one by one into the triangular prism-shaped fluorescent nucleic acid nanostructure, and as a result, it was shown that a double bond was formed at a predetermined position without writhe (FIGS. 5a and 5b). In addition, the distance between fluorescence and the graphene oxide was predicted by analyzing the data, obtained by the oxDNA simulation, using VMD (visual molecular dynamics), and as a result, it was confirmed that when the target sequences were bound, the distance between fluorescence and the graphene oxide increased (FIG. 5c).

### Example 4. Examination of Detection Ability of Structures Depending on Timing of Addition of Target Sequences

### 4-1. Synthesis of Fluorescent Nucleic Acid Nanostructure after Addition of Target Sequences

During the synthesis of the tetrahedral fluorescent nucleic acid nanostructure in Example 2-1 above, the target sequences c1, c2 and c3 were additionally added at a molar ratio of 0 to 5 times. Then, a fluorescent nucleic acid nanostructure was synthesized according to the same protocol, and the synthesis of the produced fluorescent nucleic acid nanostructure was confirmed by agarose gel electrophoresis (FIG. 6). Thereafter, 3 µg of graphene oxide was added to 20 pmol of the fluorescent nucleic acid nanostructure, and water and salt were added thereto so that the concentration of the salt reached 200 mM, thereby preparing a solution having a total volume of 50 µl. Then, the solution was allowed to react for 30 minutes. The reaction product was added to a 96-well plate and fluorescence values at absorption/emission wavelengths of FAM (485 nm/525 nm), Cy3 (540 nm/570 nm) and Cy5 (640 nm/670 nm) were measured using a plate reader. The changes in fluorescence values depending on the concentrations of the target sequence (c-DNA) were measured when the tetrahedral fluorescent nucleic acid nanostructure was at a constant concentration (20 pmol/50 µl), and as a result, it was shown that when the nanostructure was synthesized after addition of high concentrations of the target sequences, the absolute fluorescence values increased significantly, but when the nanostructure was synthesized after addition of small amounts of the target sequences, a relative difference in fluorescence between the presence and absence of the targets appeared (FIG. 7).

### 4-2. Addition of Target Sequences after Synthesis of Fluorescent Nucleic Acid Nanostructure/Graphene Oxide Complex

Target sequences were added after synthesis of a fluorescent nucleic acid nanostructure/graphene oxide complex, and the detection ability of the complex was examined. Specifically, a fluorescent nucleic acid nanostructure was synthesized as described in Example 2-1 above, and then a complex of the fluorescent nucleic acid nanostructure with graphene oxide was synthesized as described in Example 3-1 above. To 20 pmol/50 µl of the produced graphene/tetrahedral fluorescent nucleic acid nanostructure, 10 µl of each the target sequences c1, c2 and c3 (EGFR L858R, T790M and E746-A750) prepared at concentrations of 2 µM and 20 µM was added, and then how the fluorescence value increased with time was examined.

As a result, as shown in FIG. 8, it could be confirmed that, when 10-fold concentrations of the target sequences were added (FIGS. 8d to 8f), the targets did bind to the complementary sequence regions of the structure, resulting in an increase in the fluorescence values.

However, as shown in FIG. 8f, even when a 10-fold concentration of the target sequence was added, the fluorescence value was lower than that of c2. Therefore, it could be seen that when the fluorescent nucleic acid nanostructure/graphene oxide complex was formed and then reacted with the target sequences, higher concentrations of the target sequences were required than when the complex was formed after addition of the target sequences.

### Example 5. Examination of Detection Ability of Structure Depending on Salt Concentration

### 5-1. Examination of Detection Ability of Tetrahedral Fluorescent Nucleic Acid Nanostructure Depending on Salt Concentration

During synthesis of the fluorescent nucleic acid nanostructure in Example 2-1 above, the target sequences c1, c2 and c3 were added together and the fluorescent nucleic acid nanostructure was synthesized. 20 pmol of the synthesized fluorescent nucleic acid nanostructure sample was mixed with 3 µg of graphene oxide, and salt and water were added thereto so that the concentration of the salt was 0 to 200 mM, thereby preparing mixtures each having a total volume of 50 µl. Each of the mixtures was added to a 96-well plate, and fluorescence values at absorbance/emission wavelengths of 485 nm/525 nm (FAM), 540 nm/570 nm (Cy3) and 640 nm/670 nm (Cy5) were measured. As a result, it could be confirmed that, as the salt concentration increased, the fluorescence value for each target increased and it was easy to distinguish the difference between the fluorescence values (FIG. 9).

Thereby, it can be inferred that, since the surface of the graphene oxide carries a negative charge and the backbone of the nucleic acid nanostructure has a negative charge, as the salt concentration increases, the fluorescent nucleic acid nanostructure/graphene oxide complex is more easily formed, so that fluorescence attached to the single-stranded DNA is more easily quenched.

### 5-2. Examination of Detection Ability of Triangular Prism-Shaped Fluorescent Nucleic Acid Nanostructure Depending on Salt Concentration

During synthesis of the fluorescent nucleic acid nanostructure in Example 2-2 above, the target sequences c1', c2' and c3' were added together, and then the triangular prism-shaped nucleic acid nanostructure was synthesized in the same manner. During synthesis of the nanostructure, the concentration of salt was adjusted to 150 to 300 mM because the sequences were longer and more complex than those of the tetrahedral nanostructure. The synthesized fluorescent nucleic acid nanostructure sample was mixed with graphene oxide and the salt concentration was maintained at 200 mM, thereby synthesizing a graphene/triangular prism-shaped nucleic acid nanostructure. 50 µl of the synthesized graphene/triangular prism-shaped fluorescent nucleic acid nanostructure was added to a 96-well plate, and the fluorescence values at absorption/emission wavelengths of 485 nm/525 nm (FAM), 540 nm/570 nm (Cy3) and 640 nm/670 nm (Cy5) were measured using a plate reader. As a result, it could be confirmed that, when the fluorescent nucleic acid nanostructure was synthesized at a salt concentration of 150 mM, the FAM fluorescence values significantly increased even in the case of the target sequences other than the c2 sequence, but when the fluorescent nucleic acid nanostructure was synthesized at a salt concentration of 200 mM, the sensitivity for each of the targets was good (FIG. 10). In addition, even when a higher concentration of the salt was used, the target detection ability was similar to that of the nanostructure synthesized at 200 mM.

### Example 6. Examination of Detection Ability of Structures Depending on Amount of Graphene Oxide

### 6-1. Examination of Detection Ability of Tetrahedral Fluorescent Nucleic Acid Nanostructure/Graphene Oxide Depending on Amount of Graphene Oxide

During synthesis of the fluorescent nucleic acid nanostructure in Example 2-2 above, the target sequences c1, c2 and c3 were added together, and then the fluorescent nucleic acid nanostructure was synthesized in the same manner. Thereafter, using 20 pmol of the synthesized fluorescent nucleic acid nanostructure and various amounts of graphene oxide, fluorescent nucleic acid nanostructure/graphene oxide complexes were produced. Here, salt and water were further added so that the concentration of the salt was 200 mM, thereby preparing mixtures each having a total volume of 50 µl. The produced reaction products were added to a 96-well plate and the fluorescence values at absorption/emission wavelengths of FAM (485 nm/525 nm), Cy3 (540 nm/570 nm) and Cy5 (640 nm/670 nm) were measured using a plate reader. As a result, it could be confirmed that, when the amount of the graphene oxide was 2.5 to 3.5 µg, the difference in fluorescence was not significant, but as the amount of the graphene oxide increased, the FAM fluorescence signal was increasingly difficult to distinguish, and as the amount of the graphene oxide decreased, the Cy3 fluorescence signal was difficult to distinguish. Thus, it could be found that, when the amount of the graphene oxide was 3 µg, it was an appropriate amount to distinguish all fluorescence signals (FIG. 11).

### 6-2. Examination of Detection Ability of Triangular Prism-Shaped Fluorescent Nucleic Acid Nanostructure/Graphene Oxide Complex Depending on Amount of Graphene Oxide

During synthesis of the fluorescent nucleic acid nanostructure in Example 2-2 above, the target sequences c1', c2' and c3' were added together, and then the triangular prism-shaped fluorescent nucleic acid nanostructure was synthesized in the same manner. Then, using 20 pmol of the fluorescent nucleic acid nanostructure sample and various amounts (3, 6 and 9 µg) of graphene oxide, fluorescent nucleic acid nanostructure/graphene oxide complexes (graphene/fluorescent nucleic acid nanostructures) were produced. The reaction products were added to a 96 well-plate, and the fluorescence values at absorption/emission wavelengths of FAM (485 nm/525 nm), Cy3 (540 nm/570 nm) and Cy5 (640 nm/670 nm) were measured using a plate reader. As a result, it was confirmed that, when the amount of the graphene oxide was 3 µg or more, there was no significant difference in fluorescence depending on the amount of the graphene oxide (FIG. 12).

### Example 7. Detection Ability and Detection Limit of Fluorescent Nucleic Acid Nanostructure/Graphene Oxide Depending on Amount of Target Sequences

During synthesis of triangular prism-shaped nucleic acid nanostructures, the target sequences c1', c2' and c3' were added in an amount of 0 to 300 nM, and then fluorescent nucleic acid nanostructures were produced by performing the same heat-treatment protocol. Thereafter, as described in Example 3 above, 3 µg of graphene oxide was added to 10 pmol of each fluorescent nucleic acid nanostructure, and water and salt were added thereto so that the concentration of the salt reached 200 mM, thereby preparing solutions each having a total volume of 50 µl. Using the solutions, triangular prism-shaped fluorescent nucleic acid nanostructure/graphene oxide complexes were produced. Then, the fluorescence values for the complexes were measured, and standard curves depending on the amounts of target sequences added were obtained. As a result, it could be seen that the fluorescence intensities increased linearly with the concentrations of the target biomarkers (FIG. 13). In addition, the fluorescence values measured for the triangular prism-shaped fluorescent nucleic acid nanostructure/graphene oxide complexes can be converted into the concentration of the target nucleic acid biomarkers present in the sample, and thus the detection limits of the triangular prism-shaped fluorescent nucleic acid nanostructure/graphene oxide complexes were calculated. Specifically, the detection limits were calculated according to the EP17 guidelines published by the Clinical and Laboratory Standards Institute (CLSI). As a result, as shown in Table 3 below, it was found that the detection limits of the structures were 49.16 nM for miR-21 (c1'), 48.86 nM for let-7f (c2'), and 43.43 nM for miR-200b-3p (c3') (expressed in moles and grams).

**[Table 3]**

| Sequence names | Target sequences | Detection limits (nM) | Detection limits (ng/µl) |
|---|---|---|---|
| c1' | miR-21 | 49.16 | 0.3443 |
| c2' | let-7f | 48.86 | 0.3462 |
| c3' | miR-200b-3p | 43.43 | 0.3052 |

### Example 8. Examination of Function of Triangular Prism-Shaped Fluorescent Nucleic Acid Nanostructure/Graphene Oxide Complex for miRNA Derived from Lung Cancer

### 8-1. Examination of Ability to Detect miRNA Derived from Lung Cancer A549 Cells

In order to examine whether the triangular prism-shaped fluorescent nucleic acid nanostructure/graphene oxide complex of the present invention can emit fluorescence by complementary binding to a target sequence in an actual sample, the effect of the complex on the detection of miRNA extracted from lung cancer cells was examined. Specifically, total miRNA was extracted from lung cancer A549 cells, and the concentration thereof was found to be 499.68 ng/µl. During fluorescent nucleic acid nanostructures, the total miRNA extracted from the lung cancer cells was added in an amount of 0 to 10 µg, and fluorescent nucleic acid nanostructures with a concentration of 200 nM were produced. Using the nanostructures, triangular prism-shaped fluorescent nucleic acid nanostructure/graphene oxide complexes were produced. The fluorescence intensities of the produced triangular prism-shaped fluorescent nucleic acid nanostructure/graphene oxide complexes were measured, and as a result, it could be confirmed that the fluorescence intensities increased linearly with the amount of lung cancer cell-derived miRNA added (FIG. 14).

### 8-2. Examination of Ability to Detect miRNA Derived from Lung Cancer PC-9 Cells

In order to examine whether the triangular prism-shaped fluorescent nucleic acid nanostructure/graphene oxide complex of the present invention can emit fluorescence by complementary binding to a target sequence in an actual sample, the effect of the complex on the detection of miRNA extracted from lung cancer cells was examined. Specifically, total miRNA was extracted from lung cancer PC-9 cells, and the concentration thereof was found to be 312.16 ng/µl. During fluorescent nucleic acid nanostructures, the total miRNA extracted from the lung cancer cells was added in an amount of 0 to 6.24 µg, and fluorescent nucleic acid nanostructures with a concentration of 200 nM were produced. Using the nanostructures, triangular prism-shaped fluorescent nucleic acid nanostructure/graphene oxide complexes were produced. The fluorescence intensities of the produced triangular prism-shaped fluorescent nucleic acid nanostructure/graphene oxide complexes were measured, and as a result, it could be confirmed that the fluorescence intensities increased linearly with the amount of miRNA extracted from PC-9 (FIG. 15).

Thereby, it could be seen that the single-stranded nucleic acids in the prism portion of the triangular prism-shaped fluorescent nucleic acid nanostructure/graphene oxide complex of the present invention did bind complementarily to c1', c2' and c3' miRNAs present in the total miRNA of lung cancer cells, and that the fluorescence intensities increased depending on the concentrations of the target sequences.

## Claims

1. A composition for nucleic acid detection comprising: one or more single-stranded probes, each comprising a nucleic acid complementary to a target nucleic acid, a fluorescent substance, and nucleic acids complementary to other single-stranded probes; and graphene oxide.

2. The composition of claim 1, wherein the single-stranded probes are DNAs and/or RNAs.

3. The composition of claim 1, wherein the number of the single-stranded probes is 3 or more, and the composition further comprises a single-stranded probe consisting only of a nucleic acid complementary to each of the single-stranded probes.

4. The composition of claim 1, wherein the single-stranded probes comprise nucleic acids complementary to different targets for multiplex detection of different target nucleic acids.

5. The composition of claim 1, wherein, when the target nucleic acid binds to the single-stranded probe to form a double bond, the distance between the probe and the graphene oxide increases and luminescence of the fluorescent substance is detected.

6. A composition for nucleic acid detection comprising a fluorescent nucleic acid nanostructure and graphene oxide, wherein the fluorescent nucleic acid nanostructure comprises:
a first single-stranded probe comprising a nucleic acid complementary to a first target nucleic acid and a fluorescent substance;
a second single-stranded probe comprising a nucleic acid complementary to a second target nucleic acid and a fluorescent substance;
a third single-stranded probe comprising a nucleic acid complementary to a third target nucleic acid and a fluorescent substance; and
a fourth single-stranded probe consisting of a sequence complementary to the first single-stranded probe, a sequence complementary to the second single-stranded probe, and a sequence complementary to the third single-stranded probe.

7. The composition of claim 6, wherein any one of the first single-stranded probe, second single-stranded probe and third single-stranded probe of the fluorescent nucleic acid nanostructure is attached to the graphene oxide.

8. The composition of claim 7, wherein the fluorescent nucleic acid nanostructure is an inverted tetrahedral structure on the graphene oxide.

9. The composition of claim 6, wherein the first single-stranded probe, second single-stranded probe and third single-stranded probe of the fluorescent nucleic acid nanostructure are attached to the graphene oxide.

10. The composition of claim 9, wherein the fluorescent nucleic acid nanostructure is a triangular prism-shaped structure on the graphene oxide.

11. A method for nucleic acid detection comprising steps of:
isolating nucleic acid from a sample;
producing a fluorescent nucleic acid nanostructure by mixing the isolated nucleic acid with a single-stranded probe comprising a nucleic acid complementary to a target nucleic acid and a fluorescent substance, followed by heat treatment;
attaching the fluorescent nucleic acid nanostructure to graphene oxide; and
detecting fluorescence by a fluorescence resonance energy transfer phenomenon.

12. The method of claim 11, wherein multiple nucleic acids are detected using one or more single-stranded probes comprising nucleic acids complementary to different target nucleic acids and fluorescent substances.

13. A method for nucleic acid detection comprising steps of:
isolating nucleic acid from a sample;
reacting the isolated nucleic acid with the composition for nucleic acid detection according to claim 1; and
detecting fluorescence by a fluorescence resonance energy transfer phenomenon.

14. Use of a composition, which comprises one or more single-stranded probes, each comprising a nucleic acid complementary to a target nucleic acid, a fluorescent substance, and nucleic acids complementary to other single-stranded probes, and graphene oxide, for nucleic acid detection.

15. A method of detecting a biomarker using a composition comprising: one or more single-stranded probes, each comprising a nucleic acid complementary to a target nucleic acid, a fluorescent substance, and nucleic acids complementary to other single-stranded probes; and graphene oxide.
